# EUROPEAN PATENT APPLICATION

(11) **EP 4 606 323 A1**
(43) Date of publication of application: **27.08.2025**
(21) Application number: 22945633.0
(22) Date of filing: 23.12.2022
(51) Int. Cl.: A61B 17/072

(54) **SURGICAL STAPLER ASSEMBLY AND SURGICAL STAPLER**

(30) Priority: 17.10.2022 CN 202211268321; 17.10.2022 CN 202211268327
(71) Applicant: Beijing Biosis Healing Biological Technology Co., Ltd., Beijing 102600 (CN)
(72) Inventor: CHEN, Huijun, Beijing 102600 (CN); WANG, Huasheng, Beijing 102600 (CN); ZHAO, Bo, Beijing 102600 (CN); ZHAO, Yanrui, Beijing 102600 (CN)
(74) Representative: Perani & Partners S.p.A.
(86) International application number: PCT/CN2022/141306
(87) International publication number: WO 2023/236505

(57) **Abstract**

The present application provides a surgical stapler assembly, comprising: a staple cartridge assembly and an anvil assembly, which can be rotatably connected to each other. The staple cartridge assembly comprises a staple cartridge body, a staple cartridge fastener, and a staple cartridge biological patch, wherein the staple cartridge fastener can be movably connected to the staple cartridge body in a length direction of the staple cartridge body; and the staple cartridge fastener can move between a first position of the staple cartridge fastener and a second position of the staple cartridge fastener. The staple cartridge fastener is connected to the staple cartridge body in both the first position of the staple cartridge fastener and the second position of the staple cartridge fastener. When the staple cartridge fastener is in the first position of the staple cartridge fastener, the staple cartridge fastener connects a front end portion of the staple cartridge biological patch to the staple cartridge body. When the staple cartridge fastener is in the second position of the staple cartridge fastener, the staple cartridge fastener enables the front end portion of the staple cartridge biological patch to be separated from the staple cartridge body. Further provided is a surgical stapler comprising the surgical stapler assembly.

## Description

### TECHNICAL FIELD

The present application relates to the field of medical equipment, in particular to a surgical stapler assembly and a surgical stapler.

### BACKGROUND

The application of surgical staplers can significantly advance the development of modem surgery and minimally invasive surgery, marking a milestone in the field. Surgical staplers can simultaneously cut tissues and staple them using surgical staples, and are widely used for cutting, suturing, and anastomosing tissues in organs such as the intestines, stomach, lungs, and pancreas. However, relying solely on the rigid stapling of surgical staples cannot completely prevent tissue defects, leakage, or bleeding at the anastomotic site, which may lead to problems such as infection, stenosis, and scarring. Once complications such as anastomotic leakage, bleeding, stenosis, infection, and adhesions occur, a secondary surgery can be required, severely impacting the patient's quality of life and even endangering their life. This can impose significant economic and psychological burdens on the patient.

To reduce postoperative complications from anastomosis, a possible surgical stapler is provided with a reinforcing patch. Both the staple cartridge and the anvil surface of the surgical stapler are provided with a layer of absorbable reinforcing material. While performing the stapling and cutting, the reinforcing material is secured at the anastomotic site to protect fragile tissues from being torn by the surgical staples or to seal gaps between the exposed staples and the tissues or blood vessels. This reinforces the anastomotic site, thus reducing the risk of complications such as anastomotic leakage and bleeding.

In the prior art, the reinforcing patch for the anastomotic site is a sleeve-like structure, comprising a biological material layer, a backing layer, and a thread that detachably fixes the biological material layer to the backing layer. The sleeve-like structure of the anastomotic reinforcing patch is fitted over a staple cartridge portion of a staple cartridge assembly and an anvil portion of the stapler. After the reinforcing patch is secured to the tissue to be treated, the thread can be removed to detach the reinforcing patch from the backing, thereby allowing the backing to be removed.

However, using a thread to secure the biological material layer and the backing layer complicates the structure of the reinforcing patch. Special placement and fixation are required to ensure that the thread does not become tangled or disordered, which increases the number of process steps during product manufacturing, affecting production efficiency and product yield. Additionally, during surgical procedures, specific operations involving the thread, including positioning, pulling, and removal of the thread are necessary. These operations must be performed with great care to avoid errors caused by wire snagging or tissue scraping.

### SUMMARY

The present application aims to provide a surgical stapler assembly with a simplified structure, improved production efficiency, and a higher product yield.

An embodiment of the present application provides a surgical stapler assembly, comprising: a staple cartridge assembly and an anvil assembly, wherein the staple cartridge assembly and the anvil assembly are rotatably connected,
the staple cartridge assembly comprises a staple cartridge body, a staple cartridge fastener, and a staple cartridge biological patch,
the staple cartridge fastener is movably connected to the staple cartridge body along a length direction of the staple cartridge body, the staple cartridge fastener is movable between a first position of the staple cartridge fastener and a second position of the staple cartridge fastener, and the staple cartridge fastener is connected to the staple cartridge body in both the first position and the second position of the staple cartridge fastener,
when the staple cartridge fastener is in the first position of the staple cartridge fastener, the staple cartridge fastener connects a front end portion of the staple cartridge biological patch to the staple cartridge body,
when the staple cartridge fastener is in the second position of the staple cartridge fastener, the staple cartridge fastener allows the front end portion of the staple cartridge biological patch to separate from the staple cartridge body.

An embodiment of the present application further provides a surgical stapler assembly, comprising: a staple cartridge assembly and an anvil assembly, wherein the staple cartridge assembly and the anvil assembly are rotatably connected,
the anvil assembly comprises an anvil body, an anvil fastener, and an anvil biological patch,
the anvil fastener is movably connected to the anvil body along a length direction of the anvil body, the anvil fastener is movable between a first position of the anvil fastener and a second position of the anvil fastener, and the anvil fastener is connected to the anvil body in both the first position and the second position of the anvil fastener,
when the anvil fastener is in the first position of the anvil fastener, the anvil fastener connects a front end portion of the anvil biological patch to the anvil body,
when the anvil fastener is in the second position of the anvil fastener, the anvil fastener allows the front end portion of the anvil biological patch to separate from the anvil body.

In at least one alternative embodiment, the staple cartridge fastener comprises a staple cartridge engagement protrusion, and the front end portion of the staple cartridge biological patch is provided with a staple cartridge biological patch engagement hole,
when the staple cartridge fastener is in the first position of the staple cartridge fastener, the staple cartridge engagement protrusion is inserted into the staple cartridge biological patch engagement hole, such that the front end portion of the staple cartridge biological patch is connected to the staple cartridge body,
when the staple cartridge fastener is in the second position of the staple cartridge fastener, the staple cartridge engagement protrusion is withdrawn from the staple cartridge biological patch engagement hole, such that the staple cartridge biological patch can be separated from the staple cartridge body.

In at least one alternative embodiment, the staple cartridge fastener comprises an elastic retaining portion of the staple cartridge fastener,
when the staple cartridge fastener is in the first position of the staple cartridge fastener, the elastic retaining portion of the staple cartridge fastener remains in its original state or undergoes slight deformation,
when the staple cartridge fastener is in the second position of the staple cartridge fastener, the elastic retaining portion of the staple cartridge fastener is compressed to produce elastic deformation or undergoes further elastic deformation.

In at least one alternative embodiment, the anvil fastener comprises an elastic retaining portion of the anvil fastener,
when the anvil fastener is in the first position of the anvil fastener, the elastic retaining portion of the anvil fastener abuts against the anvil body to prevent the anvil fastener from moving forward,
when the anvil fastener is in the second position of the anvil fastener, the elastic retaining portion of the anvil fastener is compressed to produce elastic deformation.

In at least one alternative embodiment, the anvil fastener comprises a check portion, and the anvil body comprises a blocking portion,
when the anvil fastener is in the first position of the anvil fastener, the check portion is compressed and deformed by the blocking portion, and
when the anvil fastener is in the second position of the anvil fastener, the check portion moves to a front side of the blocking portion, such that the blocking portion can prevent the anvil fastener from moving from the second position of the anvil fastener to the first position of the anvil fastener.

In at least one alternative embodiment, the staple cartridge body is provided with a staple cartridge hook, a rear end portion of the staple cartridge biological patch is provided with a staple cartridge biological patch socket hole, the staple cartridge hook is bent backward and hooks into the staple cartridge biological patch socket hole, and/or
the anvil body is provided with an anvil hook, a rear end portion of the anvil biological patch is provided with an anvil biological patch socket hole, and the anvil hook is bent backward and hooks into the anvil biological patch socket hole.

In at least one alternative embodiment, the anvil body has a cavity, wherein a front end of the anvil body is provided with an anvil fastener mounting slot for mounting the anvil fastener, and a portion of the anvil fastener passes through the anvil fastener mounting slot and is accommodated in the cavity.

In at least one alternative embodiment, the surgical stapler assembly further comprises a cutting assembly, which is accommodated in the staple cartridge assembly and the anvil assembly,
the staple cartridge fastener and the anvil fastener can be pushed by the cutting assembly to move synchronously,
when the anvil fastener is in the first position of the anvil fastener, the staple cartridge fastener is also in the first position of the staple cartridge fastener,
when the anvil fastener is in the second position of the anvil fastener, the staple cartridge fastener is also in the second position of the staple cartridge fastener.

In at least one alternative embodiment, an anvil fastener atomizer comprises an anvil fastener engagement portion atomizer, and a front end of the anvil biological patch atomizer is provided with an anvil biological patch engagement hole atomizer,
when the anvil fastener atomizer is in the first position of the anvil fastener, the anvil fastener engagement portion atomizer is inserted into the anvil biological patch engagement hole atomizer, such that the front end of the anvil biological patch atomizer is connected to the anvil body atomizer, and
when the anvil fastener atomizer is in the second position of the anvil fastener, the anvil fastener engagement portion atomizer is withdrawn from the anvil biological patch engagement hole atomizer, such that the anvil biological patch atomizer is separated from the anvil body atomizer.

An embodiment of the present application further provides a surgical stapler assembly, comprising a staple cartridge assembly and an anvil assembly, wherein the staple cartridge assembly and the anvil assembly are rotatably connected,
the staple cartridge assembly comprises a staple cartridge body, a staple cartridge fastener, and a staple cartridge biological patch, the staple cartridge body being connected to a cutting assembly, and the cutting assembly can cut the staple cartridge biological patch,
the cutting assembly can push the staple cartridge fastener to move between a first position of the staple cartridge fastener and a second position of the staple cartridge fastener in a front-rear direction of the staple cartridge body,
when the staple cartridge fastener is in the first position of the staple cartridge fastener, the staple cartridge fastener connects a front end of the staple cartridge biological patch to the staple cartridge body,
when the staple cartridge fastener is in the second position of the staple cartridge fastener, a cutting edge or blade of the cutting assembly cuts through the staple cartridge biological patch, and the staple cartridge fastener enables the front end of the staple cartridge biological patch to separate from the staple cartridge body.

In at least one alternative embodiment, the staple cartridge fastener is movably connected to the staple cartridge body in the front-rear direction of the staple cartridge body, and the staple cartridge fastener is connected to the staple cartridge body in both the first position and the second position of the staple cartridge fastener,
the staple cartridge fastener comprises two staple cartridge engagement protrusions, the front end of the staple cartridge biological patch is provided with two staple cartridge biological patch engagement holes, and the two staple cartridge biological patch engagement holes are located on both sides of an extension line of the cutting edge or blade of the cutting assembly,
when the staple cartridge fastener is in the first position of the staple cartridge fastener, the staple cartridge engagement protrusions are inserted into the staple cartridge biological patch engagement holes, such that the front end of the staple cartridge biological patch is connected to the staple cartridge body,
when the staple cartridge fastener is in the second position of the staple cartridge fastener, the staple cartridge engagement protrusions are withdrawn from the staple cartridge biological patch engagement holes, such that the staple cartridge biological patch and the staple cartridge body are separated.

In at least one alternative embodiment, the staple cartridge fastener comprises an elastic retaining portion of the staple cartridge fastener,
when the staple cartridge fastener is in the first position of the staple cartridge fastener, the elastic retaining portion of the staple cartridge fastener remains in its original state or undergoes slight deformation, and
when the staple cartridge fastener is in the second position of the staple cartridge fastener, the elastic retaining portion of the staple cartridge fastener is compressed to produce elastic deformation or undergoes further elastic deformation.

In at least one alternative embodiment, the staple cartridge body is provided with a staple cartridge hook, a rear end portion of the staple cartridge biological patch is provided with a staple cartridge biological patch socket hole, and the staple cartridge hook hooks into the staple cartridge biological patch socket hole. The staple cartridge hook comprises two separated portions, which are located on both sides of the cutting edge or blade of the cutting assembly in a width direction of the surgical stapler assembly.

In at least one alternative embodiment, the front end of the staple cartridge biological patch is provided with a staple cartridge biological patch slot, which extends to a front end edge of the staple cartridge biological patch to form an opening. When the staple cartridge fastener is in the second position of the staple cartridge fastener, the cutting edge or blade of the cutting assembly moves into the staple cartridge biological patch slot, thereby cutting the staple cartridge biological patch.

An embodiment of the present application further provides a surgical stapler assembly, comprising a staple cartridge assembly and an anvil assembly, wherein the staple cartridge assembly and the anvil assembly are rotatably connected,
the anvil assembly comprises an anvil body, an anvil fastener, and an anvil biological patch, the anvil body being connected to a cutting assembly, and the cutting assembly being capable of cutting the anvil biological patch,
the cutting assembly is capable of pushing the anvil fastener to move between a first position of the anvil fastener and a second position of the anvil fastener in a front-rear direction of the anvil body,
when the anvil fastener is in the first position of the anvil fastener, the anvil fastener connects a front end of the anvil biological patch to the anvil body,
when the anvil fastener is in the second position of the anvil fastener, a cutting edge or blade of the cutting assembly cuts through the anvil biological patch, and the anvil fastener enables the front end of the anvil biological patch to separate from the anvil body.

In at least one alternative embodiment, the anvil fastener is movably connected to the anvil body in the front-rear direction of the anvil body, and the anvil fastener is connected to the anvil body in both the first position and the second position of the anvil fastener;
the anvil fastener comprises two anvil fastener engagement portions, the front end of the anvil biological patch is provided with two anvil biological patch engagement holes, and the two anvil biological patch engagement holes are located on both side of the extension line of the cutting edge or blade of the cutting assembly,
when the anvil fastener is in the first position of the anvil fastener, the anvil fastener engagement portions are inserted into the anvil biological patch engagement holes, such that the front end of the anvil biological patch is connected to the anvil body, and
when the anvil fastener is in the second position of the anvil fastener, the anvil fastener engagement portions are withdrawn from the anvil biological patch engagement holes, such that the anvil biological patch and the anvil body are separated.

In at least one alternative embodiment, the anvil fastener comprises an elastic retaining portion of the anvil fastener,
when the anvil fastener is in the first position of the anvil fastener, the elastic retaining portion of the anvil fastener abuts against the anvil body, thereby preventing the anvil fastener from moving forward, and
when the anvil fastener is in the second position of the anvil fastener, the elastic retaining portion of the anvil fastener is compressed to produce elastic deformation.

In at least one alternative embodiment, the anvil fastener comprises a check portion, and the anvil body comprises a blocking portion,
when the anvil fastener is in the first position of the anvil fastener, the check portion is compressed and deformed by the blocking portion, and
when the anvil fastener is in the second position of the anvil fastener, the check portion moves to a front side of the blocking portion, such that the blocking portion can prevent the anvil fastener from moving from the second position of the anvil fastener to the first position of the anvil fastener.

In at least one alternative embodiment, the anvil body is provided with an anvil hook, a rear end portion of the anvil biological patch is provided with an anvil biological patch socket hole, and the anvil hook hooks into the anvil biological patch socket hole. The anvil hook comprises two separated portions, which are located on both sides of the cutting edge or blade of the cutting assembly in the width direction of the surgical stapler assembly.

In at least one alternative embodiment, the front end of the anvil biological patch is provided with an anvil biological patch slot, which extends to a front end edge of the anvil biological patch to form an opening. When the anvil fastener is in the second position of the anvil fastener, the cutting edge or blade of the cutting assembly moves into the anvil biological patch slot, thereby cutting the anvil biological patch.

An embodiment of the present application further provides a surgical stapler, which comprises the surgical stapler assembly described in any one of the above technical solutions.

By adopting the above technical solutions, at least one of the following advantageous effects can be achieved.
(1) By connecting the front end portion of the staple cartridge biological patch to the staple cartridge body through the staple cartridge fastener, or by connecting the front end of the anvil biological patch to the anvil body through the anvil fastener, the structure of the surgical stapler assembly is simplified, the production efficiency and product yield are improved, and the operation of the surgical stapler is made easier.
(2) By enabling the front end of the staple cartridge biological patch to be cut and detached from the staple cartridge body, or by enabling the front end of the staple cartridge biological patch to be cut and detached from the staple cartridge body, there is no need to use additional tools such as scissors to cut the biological patch, thereby simplifying the surgical procedure.

### BRIEF DESCRIPTION OF THE DRAWINGS

FIG. 1 is a schematic structural view showing a surgical stapler assembly according to a first embodiment of the present application.
FIG. 2 is an exploded view showing a surgical stapler assembly according to a first embodiment of the present application.
FIG. 3 is a schematic structural view showing a staple cartridge assembly of a surgical stapler assembly according to a first embodiment of the present application.
FIGS. 4 and 5 are schematic structural views showing a staple cartridge body of a surgical stapler assembly according to a first embodiment of the present application.
FIG. 6 is a partially enlarged view showing a staple cartridge biological patch and a staple cartridge body of a surgical stapler assembly according to a first embodiment of the present application.
FIGS. 7 and 9 are schematic partial structural views showing a staple cartridge fastener of a surgical stapler assembly in a first position according to a first embodiment of the present application.
FIGS. 8 and 10 are schematic partial structural views showing a staple cartridge fastener of a surgical stapler assembly in a second position according to a first embodiment of the present application.
FIGS. 11 and 12 are schematic structural views showing a staple cartridge fastener of a surgical stapler assembly according to a first embodiment of the present application.
FIG. 13 is a schematic structural view showing an anvil assembly of a surgical stapler assembly according to a first embodiment of the present application.
FIGS. 14, 16 and 17 are schematic partial structural views showing an anvil fastener of a surgical stapler assembly in a first position according to a first embodiment of the present application.
FIGS. 15 and 18 are schematic partial structural views showing an anvil fastener of a surgical stapler assembly in a second position according to a first embodiment of the present application.
FIG. 19 is a schematic structural view showing an anvil body and an anvil biological patch of a surgical stapler assembly according to a first embodiment of the present application.
FIGS. 20 and 21 are schematic structural views showing an anvil fastener of a surgical stapler assembly according to a first embodiment of the present application.
FIG. 22 is a schematic partial structural view showing an anvil body of a surgical stapler assembly according to a first embodiment of the present application.
FIG. 23 is a schematic structural view showing a staple cartridge assembly of a surgical stapler assembly according to a second embodiment of the present application.
FIG. 24 is a schematic structural view showing a staple cartridge body of a surgical stapler assembly according to a second embodiment of the present application.
FIGS. 25 and 27 are schematic partial structural views showing a staple cartridge fastener of a surgical stapler assembly in a first position according to a second embodiment of the present application.
FIGS. 26 and 28 are schematic partial structural views showing a staple cartridge fastener of a surgical stapler assembly in a second position according to a second embodiment of the present application.
FIGS. 29 and 30 are schematic structural views showing a staple cartridge fastener of a surgical stapler assembly according to a second embodiment of the present application.
FIG. 31 is a schematic structural view showing an anvil assembly of a surgical stapler assembly according to a second embodiment of the present application.
FIG. 32 is a schematic partial structural view showing an anvil fastener of a surgical stapler assembly in a first position according to a second embodiment of the present application.
FIG. 33 is a schematic partial structural view showing an anvil fastener of a surgical stapler assembly in a second position according to a second embodiment of the present application.
FIGS. 34 and 35 are schematic structural views of an anvil fastener of a surgical stapler assembly according to a second embodiment of the present application.

### Reference List

100 staple cartridge assembly; 200 anvil assembly;
1 staple cartridge body; 11 staple cartridge hook; 12 staple cartridge fastener mounting slot; 13 limiting portion; 14 staple cartridge engagement slot; 15 inclined surface;
2 staple cartridge fastener; 21 staple cartridge fastener engagement portion; 22 staple cartridge fastener surface portion; 221 staple cartridge engagement protrusion; 23 elastic retaining portion of staple cartridge fastener;
3 staple cartridge biological patch; 31 staple cartridge biological patch engagement hole; 32 staple cartridge biological patch socket hole; 33 staple cartridge biological patch slot;
4 anvil body; 41 anvil hook; 42 anvil engagement slot; 43 anvil fastener mounting slot; 44 cavity; 45 blocking portion;
5 anvil fastener; 51 anvil fastener engagement portion; 52 elastic retaining portion of anvil fastener; 53 check portion; 54 anvil fastener body;
6 anvil biological patch; 61 anvil biological patch engagement hole; 62 anvil biological patch socket hole; 63 anvil biological patch slot;
7 slider;
8 cutting knife;
X front-rear direction; Y width direction; Z thickness direction

### DETAILED DESCRIPTION

In order to more clearly explain the above objects, features and advantages of the present application, the specific embodiments of the present application are described in detail in this section with reference to the accompanying drawings. In addition to the various embodiments described in this section, the present application can also be implemented in other different ways. Without violating the spirit of the present application, those skilled in the art can make corresponding improvements, variations and substitutions. Therefore, the present application is not limited to the specific embodiments disclosed in this section. The protection scope of the present application shall be based on the claims.

### (First Embodiment)

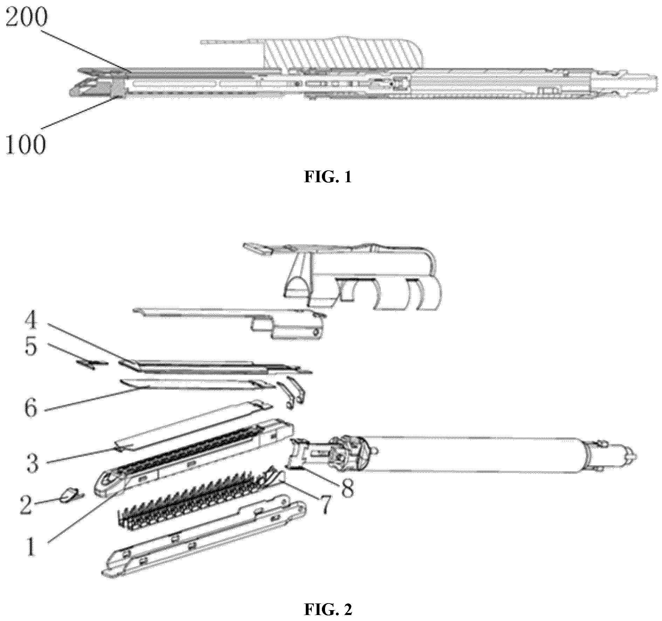

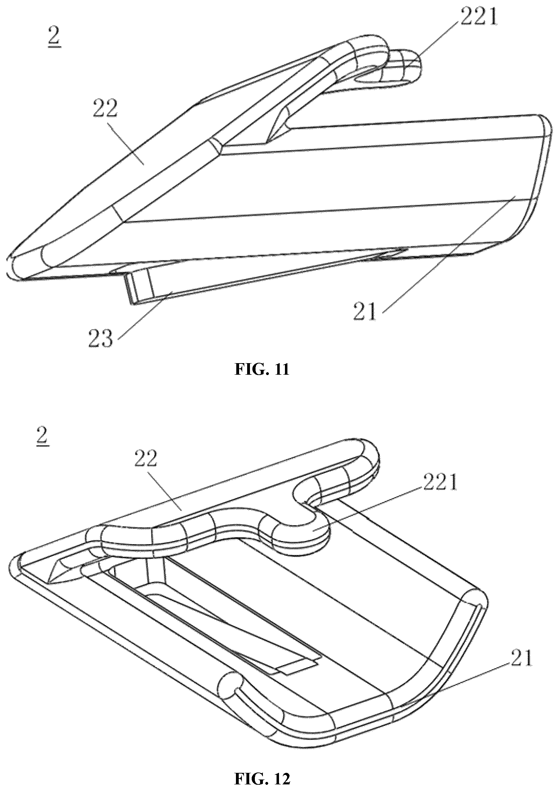

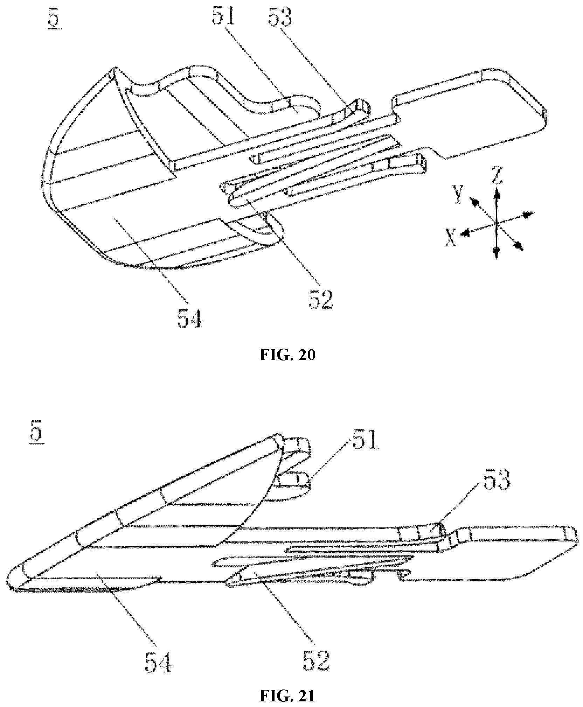

As shown in FIGS. 1 to 22, a first embodiment of the present application provides a surgical stapler, which comprises a surgical stapler assembly and a handle. The surgical stapler assembly can be located at a front end of the surgical stapler, and the handle can be located at a rear end of the surgical stapler. The surgical stapler assembly can clamp tissues of organs such as the intestines, stomach, lungs, and pancreas for cutting, suturing, and anastomosis. The surgical stapler assembly comprises a staple cartridge assembly 100 and an anvil assembly 200, which can be rotatably connected to each other so that the staple cartridge assembly 100 and the anvil assembly 200 can be opened or closed. The surgical stapler assembly further comprises a cutting assembly, which comprises a cutting knife 8 and a slider 7. The staple cartridge assembly 100 can accommodate staples and the slider 7, and the slider 7 can fire the staples when it moves forward. The slider 7 can move forward only in one direction to fire the staples, and a staple support frame supporting the staples located behind the slider 7 will block the slider 7 so that the slider 7 cannot move backward. There can be only one cutting assembly, and the cutting assembly can be connected to both the staple cartridge body 1 and the anvil body 4.

When the surgical stapler is fired, the staple cartridge assembly 100 and the anvil assembly 200 are closed, and a firing rod can move forward to push the cutting assembly in the staple cartridge assembly 100 to cut the tissue. The cutting assembly is driven to drive the slider 7 therein to move forward, and the slider 7 can fire the staples in the staple cartridge assembly 100. The legs of the staples can penetrate the tissue and press against the anvil assembly 200, where they are deformed by compression to close the wound and stop bleeding.

The cross-section of the cutting knife 8 can be in the shape of an "I". Upper and lower portions of the cutting knife 8 can be accommodated in the anvil assembly 200 and the staple cartridge assembly 100, respectively. A middle portion of the cutting knife 8 connects the upper and lower portions of the cutting knife 8. The middle portion of the cutting knife 8 can comprise a cutting edge or a blade for cutting the tissue.

### (Staple Cartridge Assembly)

As shown in FIGS. 1 to 11, the staple cartridge assembly 100 comprises a staple cartridge body 1, a staple cartridge fastener 2, a staple cartridge biological patch 3, and a slider 7. The staple cartridge body 1 is configured to accommodate staples and the slider 7. A front side of the slider 7 is provided with a wedge-shaped surface, which can push the staples out of the staple cartridge body 1. The staple cartridge fastener 2 can be slidably connected to a front end portion of the staple cartridge body 1 in a front-rear direction X with respect to the staple cartridge body 1, and front and rear ends of the staple cartridge biological patch 3 are connected to the staple cartridge body 1.

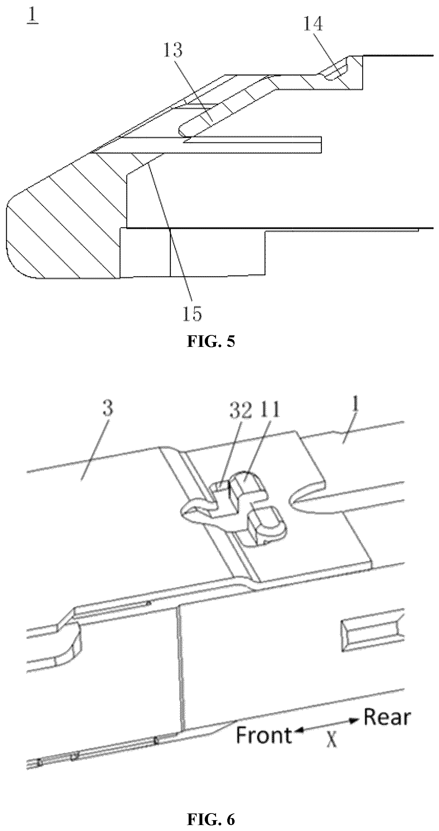

As shown in FIGS. 6 to 10, a staple cartridge biological patch engagement hole 31 is provided at the front end of the staple cartridge biological patch 3, and a staple cartridge biological patch socket hole 32 is provided at the rear end of the staple cartridge biological patch 3.

As shown in FIG. 6, a staple cartridge hook 11 is provided at the rear portion of the staple cartridge body 1, and the staple cartridge hook 11 can be bent backward to hook into the staple cartridge biological patch socket hole 32 at the rear end of the staple cartridge biological patch 3, and the rear end of the staple cartridge biological patch 3 can be connected to the staple cartridge body 1 through the staple cartridge hook 11. The staple cartridge hook 11 can comprise two separated portions, which are located on both sides of the cutting edge or blade of the cutting assembly. The cutting edge or blade of the cutting assembly can pass between the two portions to cut the staple cartridge biological patch 3 in the middle, making it easier for the staple cartridge hook 11 to separate from the divided staple cartridge biological patch 6.

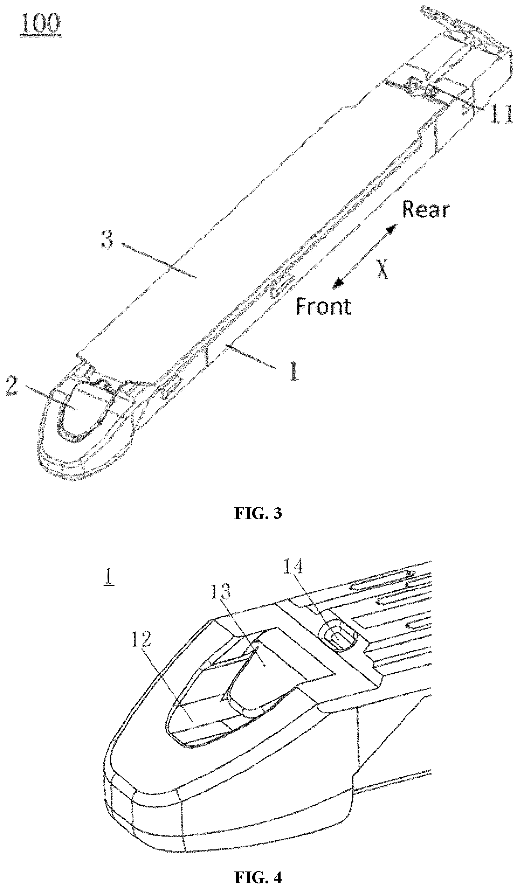

As shown in FIGS. 4, 5, 7, and 8, a staple cartridge fastener mounting slot 12 for mounting the staple cartridge fastener 2 can be formed at the front end of the staple cartridge body 1. The staple cartridge fastener mounting slot 12 can extend in the front-rear direction X, such that the staple cartridge fastener can slide along the staple cartridge fastener mounting slot 12 in the front-rear direction X.

A limiting portion 13 is further provided at the front end of the staple cartridge body 1. The limiting portion 13 can be plate-shaped and can be at least partially embedded in the staple cartridge fastener 2, such that the limiting portion 13 can limit the movement of the staple cartridge fastener 2 in a thickness direction Z thereof.

The staple cartridge body 1 is provided with a staple cartridge engagement slot 14, and the staple cartridge engagement slot 14 can be located at a rear side of the staple cartridge fastener mounting slot 12 in the front-rear direction X. The staple cartridge engagement slot 14 can accommodate a staple cartridge engagement protrusion 221 of the staple cartridge fastener 2 to be described later.

A surface of the staple cartridge body 1 facing the staple cartridge biological patch 3 can be provided with a boss, a length of which can be smaller than a length of the staple cartridge biological patch 3. The front and rear ends of the staple cartridge biological patch 3, positioned at the edges of the boss, can be bent toward the staple cartridge body 1. The staple cartridge engagement slot 14 can be located at a front side edge of the boss. Furthermore, a front side of the boss can be chamfered, and the staple cartridge engagement slot 14 can be located at the chamfer.

As shown in FIG. 5, a surface of the staple cartridge body 1 facing the elastic retaining portion 23 of the staple cartridge fastener to be described later is an inclined surface 15. When the staple cartridge fastener 2 moves forward with respect to the staple cartridge body 1, the inclined surface 15 can guide the elastic retaining portion 23 of the staple cartridge fastener to deform.

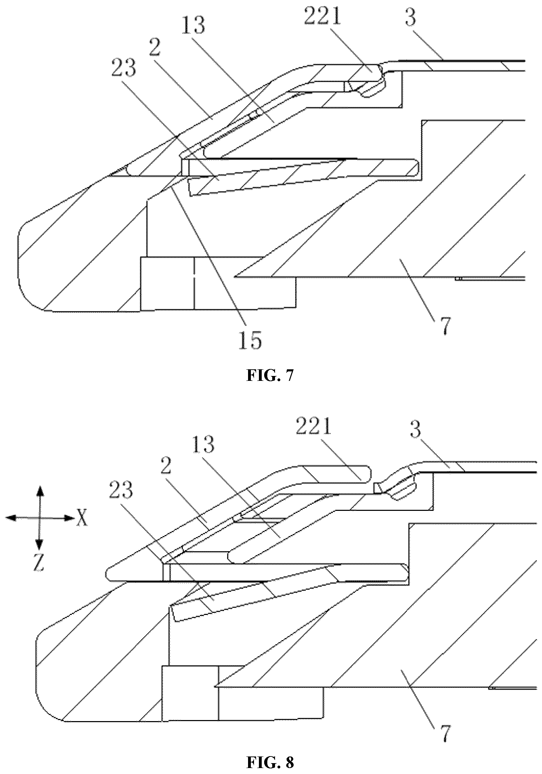

As shown in FIGS. 7 to 12, the staple cartridge fastener 2 comprises a staple cartridge fastener engagement portion 21, a staple cartridge fastener surface portion 22, and an elastic retaining portion 23 of the staple cartridge fastener.

The staple cartridge fastener engagement portion 21 and the staple cartridge fastener surface portion 22 can be fixedly connected, and the elastic retaining portion 23 of the staple cartridge fastener can be connected to the staple cartridge fastener engagement portion 21.

The cross-sectional shapes of the staple cartridge fastener engagement portion 21 and the staple cartridge fastener mounting slot 12 are the same, such that the staple cartridge fastener 2 can slide in the front-rear direction X of the staple cartridge body 1. The staple cartridge fastener 2 can be movable between a first position (also referred to as a first position of the staple cartridge fastener) and a second position (also referred to as a second position of the staple cartridge fastener), and the elastic retaining portion 23 of the staple cartridge fastener can enable the staple cartridge fastener 2 to be connected to the staple cartridge body 1 in both the first position and the second position.

As shown in FIGS. 7 to 10, the slider 7 moves to the front end of the staple cartridge body 1, and continues to move forward, which can push the staple cartridge fastener 2 to move the staple cartridge fastener 2 from the first position to the second position.

The staple cartridge fastener surface portion 22 can be located on a surface of the staple cartridge body 1 facing the anvil assembly 200, and the limiting portion 13 can be sandwiched between the staple cartridge fastener surface portion 22 and the staple cartridge fastener engagement portion 21.

A staple cartridge engagement protrusion 221 is provided at a rear side of the staple cartridge fastener surface portion 22, and the staple cartridge engagement protrusion 221 can protrude backward from the staple cartridge fastener surface portion 22.

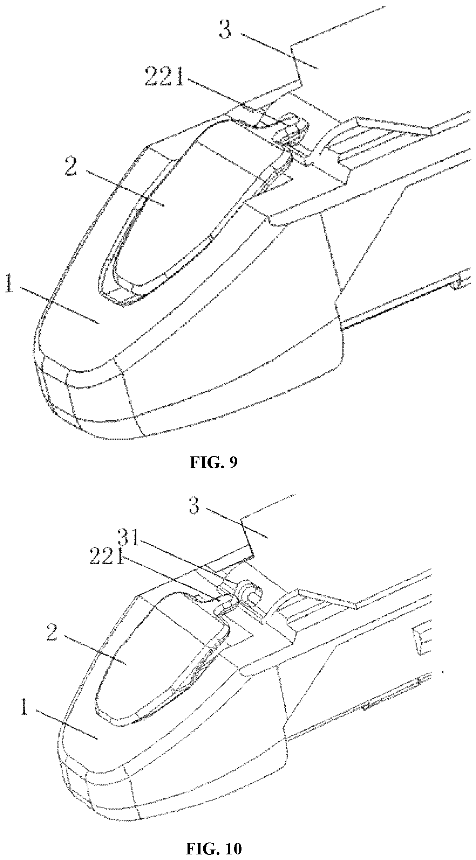

As shown in FIGS. 7 and 9, when the staple cartridge fastener 2 is in the first position, the staple cartridge engagement protrusion 221 can be at least partially inserted into the staple cartridge biological patch engagement hole 31 and the staple cartridge engagement slot 14, such that the front end of the staple cartridge biological patch 3 is connected to the staple cartridge body 1.

The elastic retaining portion 23 of the staple cartridge fastener can abut against the inclined surface 15 of the staple cartridge body 1, preventing the staple cartridge fastener 2 from freely moving forward. As a result, the staple cartridge fastener 2 can be maintained in the first position, such that the front end of the staple cartridge biological patch 3 is connected to the staple cartridge body 1. When the staple cartridge fastener 2 is in the first position, the elastic retaining portion 23 of the staple cartridge fastener can remain in its original state or undergo slight deformation.

As shown in FIGS. 8 and 10, when the staple cartridge fastener 2 is in the second position, the staple cartridge engagement protrusion 221 can be withdrawn from the staple cartridge biological patch engagement hole 31 and the staple cartridge engagement slot 14, allowing the staple cartridge biological patch 3 to separate from the staple cartridge body 1. When the staple cartridge fastener 2 is in the second position, the elastic retaining portion 23 of the staple cartridge fastener is compressed to produce elastic deformation or undergoes further elastic deformation.

The slider 7, which can move only in one direction, can prevent the staple cartridge fastener 2 from returning to the first position from the second position. This avoids re-insertion of the staple cartridge engagement protrusion 221 into the staple cartridge biological patch engagement hole 31, ensuring that the staple cartridge biological patch 3 can be detached from the staple cartridge body 1.

As shown in FIGS. 7 and 8, the elastic retaining portion 23 of the staple cartridge fastener can be deformed under an external force exerted by the slider 7. The elastic retaining portion 23 of the staple cartridge fastener 2 can engage with the inclined surface 15 of the staple cartridge body 1, making it difficult for the staple cartridge fastener 2 to move forward continuously and disengage from the staple cartridge body 1.

### (Anvil Assembly)

As shown in FIGS. 1, 2, and 13 to 22, the anvil assembly 200 comprises an anvil body 4, an anvil fastener 5, and an anvil biological patch 6. When the surgical stapler is fired, the staples can penetrate the staple cartridge biological patch 3 and the anvil biological patch 6 and press against the anvil body 4, where they are compressed and deformed to close the wound and stop bleeding. The front and rear ends of the anvil biological patch 6 are connected to the anvil body 4. The anvil fastener 5 can be connected to the front end portion of the anvil body 4. The anvil fastener 5 can be located on the front side of the anvil biological patch 6, and the front end of the anvil biological patch 6 is connected to the anvil body 4 through the anvil fastener 5.

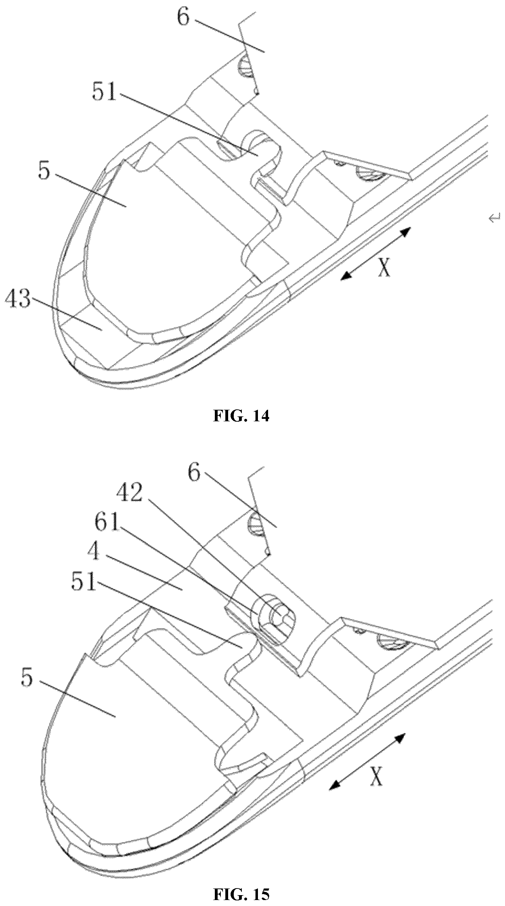

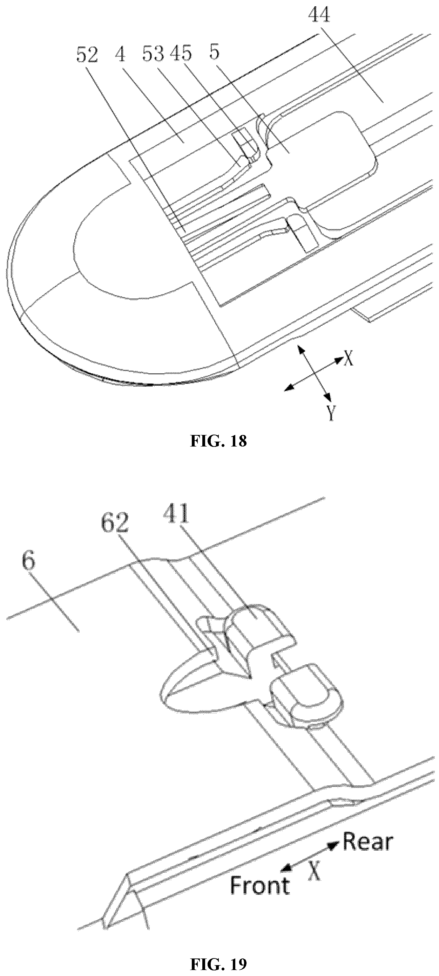

As shown in FIGS. 15 and 19, an anvil biological patch engagement hole 61 is provided at the front end of the anvil biological patch 6, and an anvil biological patch socket hole 62 is provided at the rear end of the anvil biological patch 6.

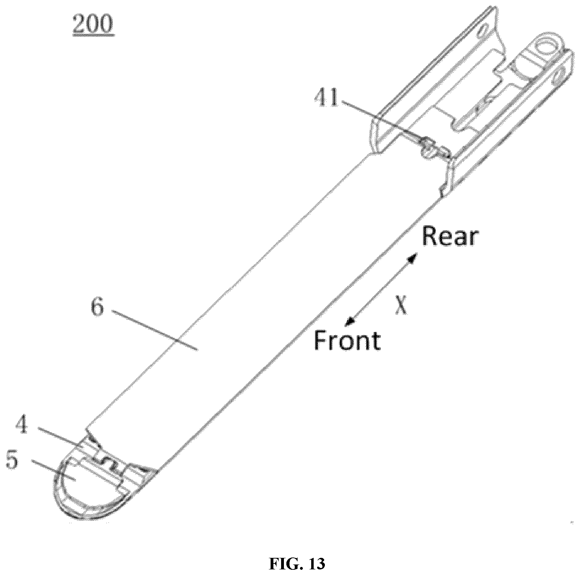

As shown in FIGS. 13 and 19, an anvil hook 41 is provided at a rear portion of the anvil body 4, and the anvil hook 41 can be bent backward to hook into the anvil biological patch socket hole 62 at the rear end of the anvil biological patch 6. The rear end of the anvil biological patch 6 can be connected to the anvil body 4 through the anvil hook 41. The anvil hook 41 can comprise two separated portions. The cutting edge or blade of the cutting assembly can pass between the two portions to cut the anvil biological patch 6 in the middle, making it easier for the anvil hook 41 to separate from the divided anvil biological patch 6.

As shown in FIGS. 14 to 18 and FIG. 22, an anvil fastener mounting slot 43 for mounting the anvil fastener 5 can be formed at the front end of the anvil body 4. The anvil fastener mounting slot 43 can extend in the front-rear direction X, such that the anvil fastener 5 can slide along the anvil fastener mounting slot in the front-rear direction X.

A cavity 44 can be formed in the anvil body 4. The anvil fastener mounting slot 43 can extend through a front side wall of the cavity 44, such that a portion of the anvil fastener 5 is accommodated in the cavity 44.

As shown in FIG. 15, the anvil body 4 is provided with an anvil engagement slot 42, which can accommodate an anvil fastener engagement portion 51 of the anvil fastener 5 to be described later.

Furthermore, a boss can be provided on a surface of the anvil body 4 facing the anvil biological patch 6. A length of the boss can be smaller than that of the anvil biological patch 6. The front and rear ends of the anvil biological patch 6, positioned at the edges of the boss, can be bent toward the anvil body 4. The anvil engagement slot 42 can be located at a front side edge of the boss. Further, a front side of the boss can be chamfered, and the anvil engagement slot 42 can be located at the chamfer.

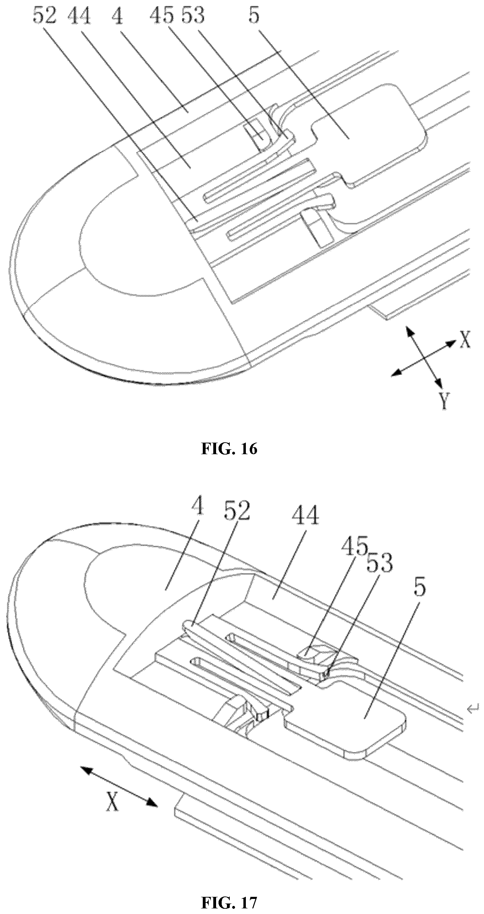

As shown in FIGS. 16 to 18, the cavity 44 of the anvil body 4 can be provided with a blocking portion 45. There can be two blocking portions 45, which can be arranged on two opposite side walls of the cavity 44, respectively. The two blocking portions 45 are spaced apart in a width direction Y of the anvil body 4. The portion of the anvil fastener 5 within the cavity 44 is located between the two blocking portions 45, and the blocking portions 45 can contact and fit with the anvil fastener 5.

As shown in FIGS. 14 to 21, the anvil fastener 5 comprises an anvil fastener engagement portion 51, an elastic retaining portion 52 of the anvil fastener, a check portion 53, and an anvil fastener body 54.

The anvil fastener engagement portion 51 can protrude backward from the anvil fastener body 54, facilitating insertion into the anvil biological patch engagement hole 61 and the anvil engagement slot 42. When the anvil fastener 5 is in the first position, the anvil fastener engagement portion 51 can be inserted into the anvil biological patch engagement hole 61 and the anvil engagement slot 42.

The elastic retaining portion 52 of the anvil fastener can be connected to the anvil fastener body 54, and the elastic retaining portion 52 of the anvil fastener can extend forward from the anvil fastener body 54. During the process of inserting the anvil fastener 5 into the anvil fastener mounting slot 43 of the anvil body 4, the elastic retaining portion 52 of the anvil fastener can be compressed in its thickness direction Z. When the anvil fastener 5 is mounted onto the anvil body 4 and is in the first position, the elastic retaining portion 52 of the anvil fastener can completely pass through the anvil fastener mounting slot 43, releasing its deformation.

The check portion 53 can be connected to the anvil fastener body 54. There can be two check portions 53, which can extend from the anvil fastener body 54 to both sides in a width direction Y thereof. The check portions 53 can be elastically deformed when compressed, which narrows a distance between the two check portions 53. During the process of inserting the anvil fastener 5 into the anvil fastener mounting slot 43 of the anvil body 4, the check portions 53 can be compressed in the width direction Y thereof. When the anvil fastener 5 is mounted onto the anvil body 4 and is in the first position, the check portions 53 can be blocked by the blocking portions 45 and remain in a compressed state.

The elastic retaining portion 52 of the anvil fastener and the check portions 53 can pass through the anvil fastener mounting slot 43 after elastic deformation, such that the anvil fastener 5 is mounted to the anvil body 4. The elastic retaining portion 52 of the anvil fastener can abut against the wall of the cavity 44, thereby preventing the anvil fastener 5 from separating from the anvil body 4. The anvil fastener 5 can be connected to the anvil body 4 in both the first position (also referred to as the first position of the anvil fastener) and the second position (also referred to as the second position of the anvil fastener) which will be described later.

As shown in FIGS. 14, 16, and 17, when the anvil fastener 5 is in the first position, the check portions 53 cooperate with the blocking portions 45, and the check portions 53 can be compressed by the blocking portions 45 and elastically deformed. The elastic retaining portion 52 of the anvil fastener can abut against the front side wall of the cavity 44, preventing the anvil fastener 5 from moving forward when no external force is applied.

During the process of the anvil fastener 5 moving forward from the first position to the second position, the anvil fastener 5 can be pushed by the cutting assembly. The elastic retaining portion 52 of the anvil fastener can undergo elastic deformation but still abut against the front side wall of the cavity 44, preventing the anvil fastener 5 from exiting the cavity 44 and separating from the anvil body 4.

As shown in FIGS. 15 and 18, when the anvil fastener 5 is in the second position, after the check portions 53 have completely passed the blocking portions 45, the check portions 53 are no longer compressed and can return to their original state. As a result, the blocking portions 45 can block the check portions 53 from moving backward. This can prevent the anvil fastener 5 from returning to the first position from the second position, and further prevent the anvil fastener engagement portion 51 from being inserted into the anvil biological patch engagement hole 61 again, which makes it impossible for the anvil biological patch 6 to be detached from the anvil body 4.

Under the linkage action of the cutting assembly, the anvil fastener 5 and the staple cartridge fastener 2 can be fired to move synchronously. When the anvil fastener 5 is in the first position, the staple cartridge fastener 2 is also in the first position; and when the anvil fastener 5 is in the second position, the staple cartridge fastener 2 is also in the second position.

The staple cartridge biological patch 3 and the anvil biological patch 6 are made of in vivo degradable polymer materials or in vivo degradable biological materials. The polymer materials can include polypropylene, polylactic acid (PLA), polyglycolic acid (PGA), or poly(lactic-co-glycolic acid) (PLGA), which have good compatibility with tissues. The in vivo degradable biological materials include dermal matrix, pericardium, peritoneum, bladder submucosa, or small intestinal submucosa that has undergone immunogen removal treatment, and preferably include decellularized small intestinal submucosa matrix material.

The staple cartridge biological patch 3 and the anvil biological patch 6 can be used in a variety of clinical surgeries for soft tissue repair. After the staples are fired, the staple cartridge biological patch 3 and the anvil biological patch 6 can be fixed to the tissue by the staples. The staple cartridge biological patch 3 and the anvil biological patch 6 can proactively induce cell ingrowth, enabling in vivo degradation and tissue regeneration. This upgrades replacement repair to regenerative repair traditional surgery, while also protecting the tissue from being torn by the staples and sealing the gaps between the exposed staples and the tissue or blood vessels. This reduces the risk of postoperative complications, allowing patients to achieve better recovery and enjoy an improved quality of life after surgery.

The surgical stapler assembly of the present application is connected to the staple cartridge biological patch 3 and the anvil biological patch 6, featuring a simple structure that offers high production efficiency and product yield. After the cutting assembly cuts the tissue, the staple cartridge biological patch 3 and the anvil biological patch 6 can be easily detached without complex operations, avoiding risks caused by wire snagging, tissue scraping, or the like during the surgery.

Below is an introduction to the working process of using the surgical stapler.

The staple cartridge assembly 100 and the anvil assembly 200 clamp the tissue to be cut. When the surgical stapler is fired, the cutting assembly moves forward to cut the tissue and pushes the slider 7 to fire the staples. The cutting assembly cuts the staple cartridge biological patch 3 and the anvil biological patch 6 in the middle. Additionally, the slider 7 fires the staples so that they pass through the tissue and are pressed against the anvil assembly 200, where they are compressed and deformed to close the wound and stop bleeding. When the staple cartridge biological patch 3 and the anvil biological patch 6 are cut by the cutting assembly, the staple cartridge hook 11 can easily disengage from the staple cartridge biological patch socket hole 32, and the anvil hook 41 can easily disengage from the anvil biological patch socket hole 62. As the cutting assembly drives the slider 7 to move forward, the staple cartridge biological patch 3 and the anvil biological patch 6 can be fixed to the tissue by the staples. The slider 7 pushes the staple cartridge fastener 2, causing the staple cartridge engagement protrusion 221 to withdraw from the staple cartridge biological patch engagement hole 31, thereby separating the front end of the staple cartridge biological patch 3 from the staple cartridge body 1. The cutting assembly pushes the anvil fastener 5, causing the anvil fastener engagement portion 51 to withdraw from the anvil biological patch engagement hole 61, thereby separating the front end of the anvil biological patch 6 from the anvil body 4. As a result, the staple cartridge biological patch 3 and the anvil biological patch 6, which are stapled to the tissue, are detached from the surgical stapler.

### (Second Embodiment)

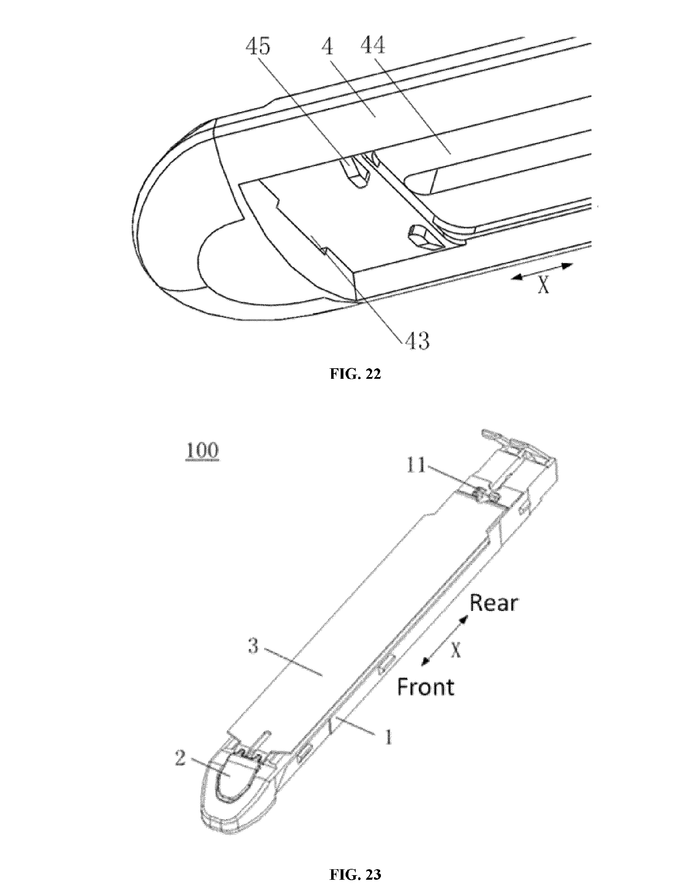

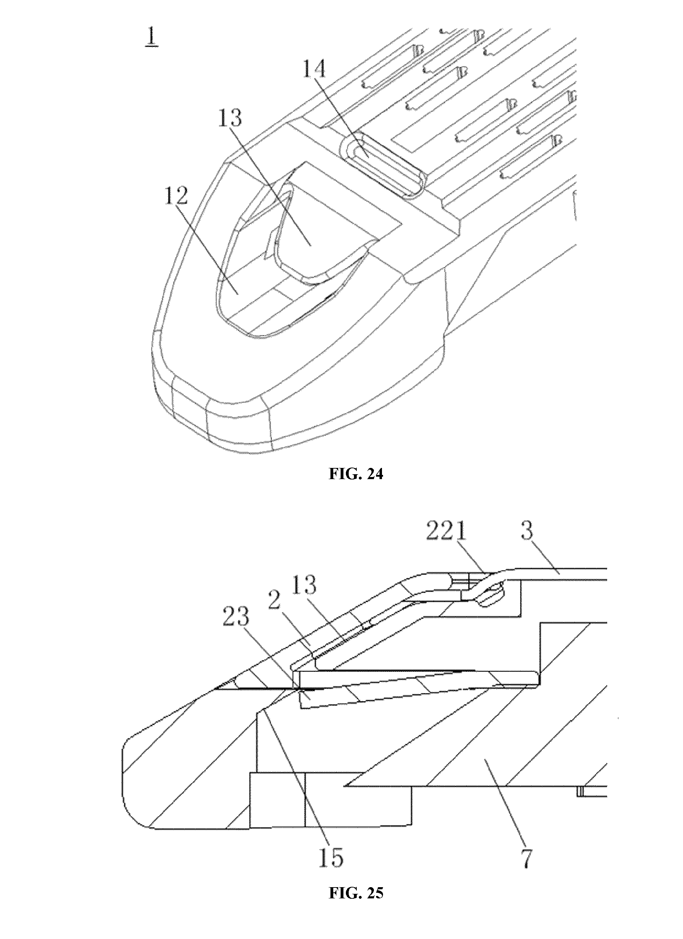

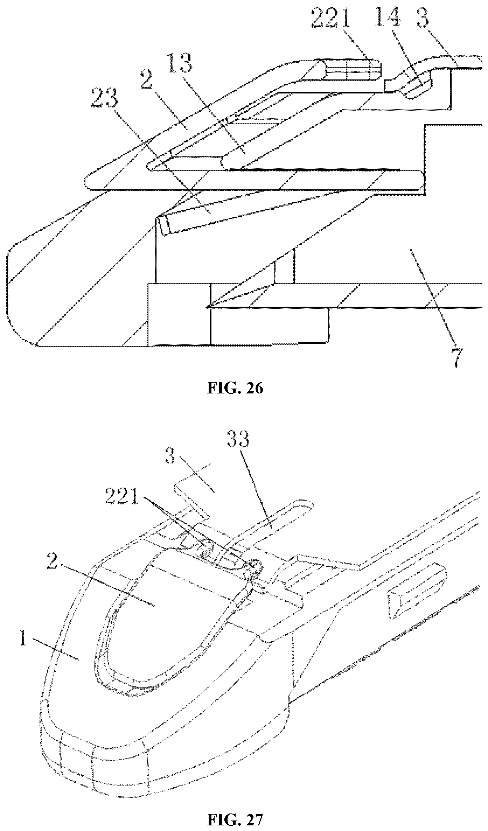

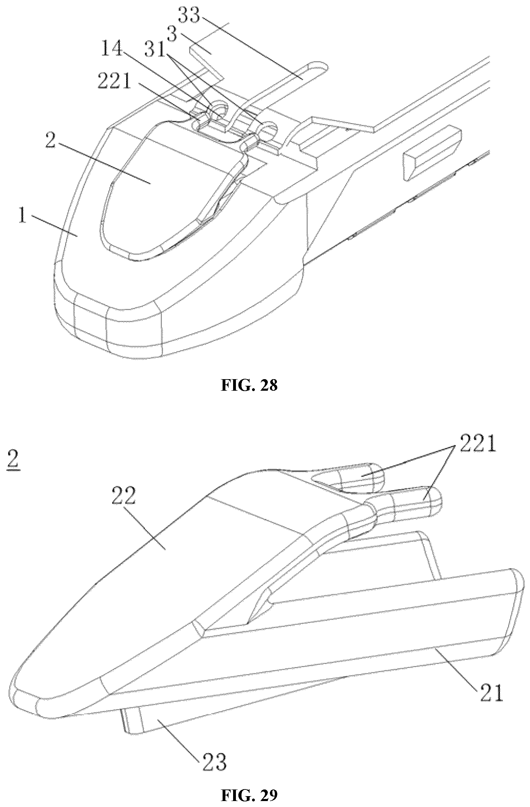

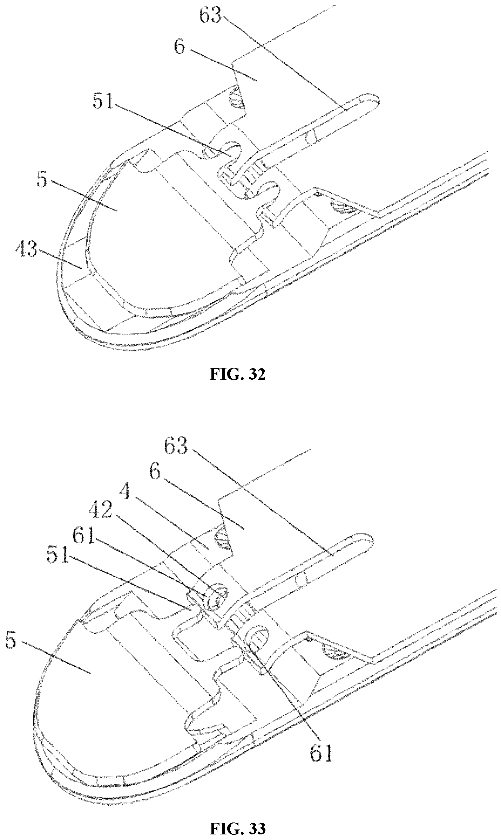

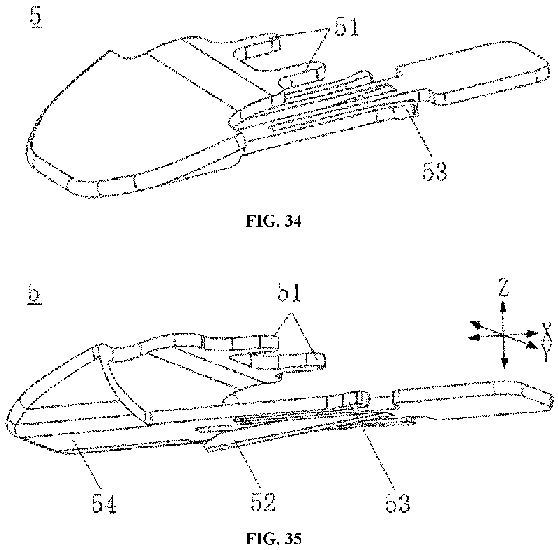

As shown in FIGS. 23 to 35, a second embodiment of the present application provides a surgical stapler. The same reference signs are used to denote the same or similar components of the surgical stapler of the second embodiment as those of the surgical stapler of the first embodiment.

The differences between the surgical stapler of the second embodiment and the surgical stapler of the first embodiment include the staple cartridge engagement protrusion 221, the staple cartridge biological patch 3, the anvil fastener engagement portion 51, and the anvil biological patch 6.

### (Staple Cartridge Assembly)

As shown in FIGS. 23, 27, and 28, the front end of the staple cartridge biological patch 3 is provided with a staple cartridge biological patch engagement hole 31 and a staple cartridge biological patch slot 33.

The staple cartridge biological patch slot 33 can extend in the front-rear direction X. The staple cartridge biological patch slot 33 extends to a front end edge of the staple cartridge biological patch 3 to form an opening. The staple cartridge biological patch slot 33 can correspond to the position of the cutting edge or blade of the cutting assembly. For example, the staple cartridge biological patch slot 33 is located in the middle of the staple cartridge biological patch 3 in the width direction Y. A rear end, or a bottom portion, of the staple cartridge biological patch slot 33 extends to a rear side of the staple cartridge biological patch engagement hole 31. Here, by way of example, a length of the staple cartridge biological patch slot 33 can be 3 times or more, or 5 times or more, a diameter of the staple cartridge biological patch engagement hole 31. The length of the staple cartridge biological patch slot 33 can be less than or equal to one-third or one-fifth of the length of the staple cartridge biological patch 3 in the front-rear direction X. The staple cartridge biological patch slot 33 with an appropriate length not only enables the cutting edge or blade of the cutting assembly to cut off the staple cartridge biological patch 3, but also enables the staple cartridge biological patch 3 to have certain stiffness or hardness, preventing it from being too soft and prone to positional deviation.

There can be two staple cartridge biological patch engagement holes 31, which are separated by the staple cartridge biological patch slot 33. The two staple cartridge biological patch engagement holes 31 are arranged on both sides of the staple cartridge biological patch slot 33 (that is, the extension line of the cutting edge or blade of the cutting assembly). The staple cartridge biological patch slot 33 helps to completely cut the staple cartridge biological patch 3 into two parts. When the cutting assembly cuts from the rear side to the front side of the staple cartridge biological patch 3 until it reaches the bottom portion of the staple cartridge biological patch slot 33, the staple cartridge biological patch 3 can be divided into two.

There can be two staple cartridge engagement protrusions 221. The two staple cartridge engagement protrusions 221 are located on both sides of the cutting edge or blade of the cutting assembly, respectively, and can be inserted into the two staple cartridge biological patch engagement holes 31.

The staple cartridge engagement slot 14 can accommodate the two staple cartridge engagement protrusions 221.

When the staple cartridge fastener 2 is pushed by the cutting assembly and is in the second position, the cutting knife cuts from the rear to the front until it reaches the bottom portion of the staple cartridge biological patch slot 33. The cutting assembly can directly and completely cut off the staple cartridge biological patch 3, eliminating the need to use additional tools such as scissors to cut the staple cartridge biological patch 3. Thus, the surgical stapler can be used in narrow operating spaces, reducing the size of surgical incisions, for example, making it suitable for endoscopic surgeries.

The slider 7, which can move only in one direction, can prevent the staple cartridge fastener 2 from returning to the first position from the second position. This avoids re-insertion of the staple cartridge engagement protrusion 221 into the staple cartridge biological patch engagement hole 31, ensuring that the staple cartridge biological patch 3 can be detached from the staple cartridge body 1.

### (Anvil Assembly)

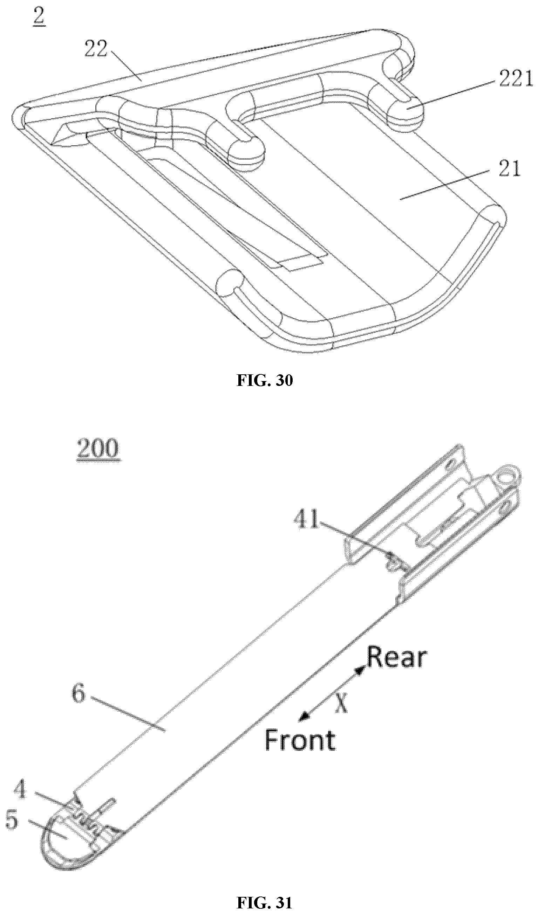

As shown in FIGS. 31 to 35, the front end of the anvil biological patch 6 is provided with an anvil biological patch engagement hole 61 and an anvil biological patch slot 63.

The anvil biological patch slot 63 can extend in the front-rear direction X. The anvil biological patch slot 63 extends to a front end edge of the anvil biological patch 6 to form an opening. The anvil biological patch slot 63 can correspond to the position of the cutting edge or blade of the cutting assembly. For example, the anvil biological patch slot 63 is located in the middle of the anvil biological patch 6 in the width direction Y. A rear end, or a bottom portion, of the anvil biological patch slot 63 extends to a rear side of the anvil biological patch engagement hole 61. By way of example, a length of the anvil biological patch slot 63 can be 3 times or more, or 5 times or more, a diameter of the anvil biological patch engagement hole 61. The length of the anvil biological patch slot 63 can be less than or equal to one-third or one-fifth of the length of the anvil biological patch 6 in the front-rear direction X. The anvil biological patch slot 63 with an appropriate length not only enables the cutting edge or blade of the cutting assembly to cut off the anvil biological patch 6, but also enables the anvil biological patch 6 to have certain stiffness or hardness, preventing it from being too soft and prone to positional deviation.

There can be two anvil biological patch engagement holes 61, which are separated by the anvil biological patch slot 63. The two anvil biological patch engagement holes 61 are located on both sides of the anvil biological patch slot 63 (that is, the extension line of the cutting edge or blade of the cutting assembly). The anvil biological patch slot 63 helps to completely cut the anvil biological patch 6 into two parts. When the cutting assembly cuts from the rear side to the front side of the anvil biological patch 6 until it reaches the bottom portion of the anvil biological patch slot 63, the anvil biological patch 6 can be divided into two.

The shapes of the anvil biological patch 6 and the staple cartridge biological patch 3 can be the same, so that the anvil biological patch 6 and the staple cartridge biological patch 3 can be mounted interchangeably.

There can be two anvil fastener engagement portions 51. The two anvil fastener engagement portions 51 are located on both sides of the cutting edge or blade of the cutting assembly, respectively, and can be inserted into the two anvil biological patch engagement holes 61, respectively.

The anvil engagement slot 42 can accommodate the two anvil fastener engagement portions 51.

When the anvil fastener 5 is pushed by the cutting assembly and is in the second position, the cutting knife cuts from the rear to the front until it reaches the bottom portion of the anvil biological patch slot 63. The cutting assembly can directly and completely cut off the anvil biological patch 6, eliminating the need to use additional tools such as scissors to cut the anvil biological patch 6.

The positions, quantities, shapes, etc. of the anvil biological patch slots 63 and the anvil biological patch engagement holes 61 described herein are merely exemplary and not restrictive. For example, only one anvil biological patch engagement hole 61 can be provided. Alternatively, the anvil biological patch slot 63 can extend in a curved manner rather than in a straight line. Alternatively, one of the anvil biological patch slot 63 and the anvil biological patch engagement hole 61 can be located in the middle of the anvil biological patch 6 in the width direction Y, and the other can be located on one side of the one in the width direction Y.

Likewise, the positions, quantities, shapes, etc. of the staple cartridge biological patch slots 33 and staple cartridge biological patch engagement holes 31 described herein are merely exemplary and not restrictive. The shapes, structures, etc. of the anvil biological patch 6 and the staple cartridge biological patch 3 can be the same or different.

Although the present application has been described in detail using the above embodiments, the following explanations are provided.
(I) In the above embodiments, the staple cartridge assembly 100 comprises the staple cartridge biological patch 3, and the anvil assembly 200 comprises the anvil biological patch 6, but the present application is not limited thereto. It is possible for only one of the staple cartridge assembly and the anvil assembly to comprise a biological patch, that is, the staple cartridge assembly comprises the staple cartridge biological patch or the anvil assembly comprises the anvil biological patch.
(II) The front side wall of the cavity 44 can be formed into an inclined surface or a curved surface. During the movement of the anvil fastener from the first position to the second position, the inclined surface or the curved surface is beneficial for guiding the elastic deformation of the elastic retaining portion 52 of the anvil fastener.

It is appreciated that in the present application, when the number of a part or component is not specifically defined, the number can be one or more, where "more" refers to two or more. In cases where the drawings show and/or the description indicates a specific number of parts or components, such as two, three, or four, this specific number is usually exemplary rather than restrictive and can be understood as "more", that is, two or more. However, this does not mean that the present application excludes the case of one.

In the present application, unless otherwise explicitly stated or defined, terms such as "install", "assembly", "mount", "connect", "link", "couple", "join", "abut", "communicate", "intercommunicate", "conduct", "fix", and "fasten" should be interpreted in a broad sense. For example, these terms can refer to direct or indirect relationships. For instance, regarding the connect, it can be fixed connection, detachable connection, or integral connection; it can be mechanical connection or electrical connection; it can be direct connection or indirect connection through an intermediary medium; and it can refer to internal communication between two components or interaction between two components, unless otherwise explicitly stated or defined. For example, regarding communication/conduction, it can be direct communication/conduction or indirect communication/conduction through an intermediary medium. For those of ordinary skill in the art, they can understand the specific meanings of the above terms in the present application based on the specific circumstances.

In the present application, unless otherwise explicitly stated or defined, when a component is provided/mounted/located/accommodated/placed in, within, or inside another component, it can be either of the following two situations: a portion or a substantial portion of the component is located inside the another component; and the component is completely accommodated inside the another component.

Although the present application has been described in detail using the above embodiments, it is obvious to those skilled in the art that the present application is not limited to the embodiments described herein. The present application can be modified and implemented as variant embodiments without departing from the spirit and scope of the present application as defined by the claims. Therefore, the descriptions herein are for illustrative purposes only and do not have any limiting implications for the present application.

## Claims

1. A surgical stapler assembly, comprising a staple cartridge assembly (100) and an anvil assembly (200), the staple cartridge assembly (100) and the anvil assembly (200) being rotatably connected, **characterized in that**,
the staple cartridge assembly (100) comprises a staple cartridge body (1), a staple cartridge fastener (2), and a staple cartridge biological patch (3),
the staple cartridge fastener (2) is movably connected to the staple cartridge body (1) in a length direction (X) of the staple cartridge body (1), the staple cartridge fastener (2) is movable between a first position of the staple cartridge fastener and a second position of the staple cartridge fastener, and the staple cartridge fastener (2) is connected to the staple cartridge body (1) in both the first position of the staple cartridge fastener and the second position of the staple cartridge fastener,
when the staple cartridge fastener (2) is in the first position of the staple cartridge fastener, the staple cartridge fastener (2) connects a front end portion of the staple cartridge biological patch (3) to the staple cartridge body (1),
when the staple cartridge fastener (2) is in the second position of the staple cartridge fastener, the staple cartridge fastener (2) enables the front end portion of the staple cartridge biological patch (3) to be separated from the staple cartridge body (1).

2. A surgical stapler assembly, comprising a staple cartridge assembly (100) and an anvil assembly (200), the staple cartridge assembly (100) and the anvil assembly (200) being rotatably connected, **characterized in that**,
the anvil assembly (200) comprises an anvil body (4), an anvil fastener (5), and an anvil biological patch (6),
the anvil fastener (5) is movably connected to the anvil body (4) in a length direction (X) of the anvil body (4), the anvil fastener (5) is movable between a first position of the anvil fastener and a second position of the anvil fastener, and the anvil fastener (5) is connected to the anvil body (4) in both the first position of the anvil fastener and the second position of the anvil fastener,
when the anvil fastener (5) is in the first position of the anvil fastener, the anvil fastener (5) connects a front end portion of the anvil biological patch (6) to the anvil body (4),
when the anvil fastener (5) is in the second position of the anvil fastener, the anvil fastener (5) enables the front end portion of the anvil biological patch (6) to be separated from the anvil body (4).

3. The surgical stapler assembly according to claim 1, **characterized in that**, the staple cartridge fastener (2) comprises a staple cartridge engagement protrusion (221), and a staple cartridge biological patch engagement hole (31) is provided at the front end portion of the staple cartridge biological patch (3),
when the staple cartridge fastener (2) is in the first position of the staple cartridge fastener, the staple cartridge engagement protrusion (221) is inserted into the staple cartridge biological patch engagement hole (31), whereby the front end portion of the staple cartridge biological patch (3) is connected to the staple cartridge body (1),
when the staple cartridge fastener (2) is in the second position of the staple cartridge fastener, the staple cartridge engagement protrusion (221) is withdrawn from the staple cartridge biological patch engagement hole (31), whereby the staple cartridge biological patch (3) and the staple cartridge body (1) can be separated.

4. The surgical stapler assembly according to claim 2, **characterized in that**, the anvil fastener (5) comprises an elastic retaining portion (52) of the anvil fastener,
when the anvil fastener (5) is in the first position of the anvil fastener, the elastic retaining portion (52) of the anvil fastener abuts against the anvil body (4) to prevent the anvil fastener (5) from moving forward,
when the anvil fastener (5) is in the second position of the anvil fastener, the elastic retaining portion (52) of the anvil fastener is compressed to produce elastic deformation.

5. The surgical stapler assembly according to claim 2, **characterized in that** the anvil fastener (5) comprises a check portion, and the anvil body (4) comprises a blocking portion,
when the anvil fastener (5) is in the first position of the anvil fastener, the check portion is compressed and deformed by the blocking portion,
when the anvil fastener (5) is in the second position of the anvil fastener, the check portion moves to a front side of the blocking portion, whereby the blocking portion can prevent the anvil fastener (5) from moving from the second position of the anvil fastener to the first position of the anvil fastener.

6. The surgical stapler assembly according to claim 1 or 2, **characterized in that**,
the staple cartridge body (1) is provided with a staple cartridge hook (11), a rear end portion of the staple cartridge biological patch (3) is provided with a staple cartridge biological patch socket hole (32), the staple cartridge hook (11) is bent backward and hooks into the staple cartridge biological patch socket hole (32), and/or
the anvil body (4) is provided with an anvil hook (41), a rear end portion of the anvil biological patch (6) is provided with an anvil biological patch socket hole (62), and the anvil hook (41) is bent backward and hooks into the anvil biological patch socket hole (62).

7. The surgical stapler assembly according to claim 2, **characterized in that**, the anvil body (4) has a cavity (44), a front end of the anvil body (4) is provided with an anvil fastener mounting slot (43) for mounting the anvil fastener (5), and a portion of the anvil fastener (5) passes through the anvil fastener mounting slot (43) and is accommodated in the cavity (44).

8. The surgical stapler assembly according to claim 1 or 2, **characterized in that**, the surgical stapler assembly further comprises a cutting assembly, the cutting assembly is accommodated in the staple cartridge assembly (100) and the anvil assembly (200),
the staple cartridge fastener (2) and the anvil fastener (5) can be pushed by the cutting assembly to move synchronously,
when the anvil fastener (5) is in the first position of the anvil fastener, the staple cartridge fastener (2) is in the first position of the staple cartridge fastener,
when the anvil fastener (5) is in the second position of the anvil fastener, the staple cartridge fastener (2) is in the second position of the staple cartridge fastener.

9. The surgical stapler assembly according to claim 2, **characterized in that**,
the anvil fastener (5) comprises an anvil fastener engagement portion (51), and the front end of the anvil biological patch (6) is provided with an anvil biological patch engagement hole (61),
when the anvil fastener (5) is in the first position of the anvil fastener, the anvil fastener engagement portion (51) is inserted into the anvil biological patch engagement hole (61), whereby the front end of the anvil biological patch (6) is connected to the anvil body (4), and
when the anvil fastener (5) is in the second position of the anvil fastener, the anvil fastener engagement portion (51) is withdrawn from the anvil biological patch engagement hole (61), whereby the anvil biological patch (6) and the anvil body (4) can be separated.

10. A surgical stapler assembly, comprising a staple cartridge assembly (100) and an anvil assembly (200), the staple cartridge assembly (100) and the anvil assembly (200) being rotatably connected, **characterized in that**,
the staple cartridge assembly (100) comprises a staple cartridge body (1), a staple cartridge fastener (2), and a staple cartridge biological patch (3), the staple cartridge body (1) being connected to a cutting assembly, and the cutting assembly can cut the staple cartridge biological patch (3),
the cutting assembly can push the staple cartridge fastener (2) in a front-rear direction (X) of the staple cartridge body (1) to move between a first position of the staple cartridge fastener and a second position of the staple cartridge fastener,
when the staple cartridge fastener (2) is in the first position of the staple cartridge fastener, the staple cartridge fastener (2) connects a front end of the staple cartridge biological patch (3) to the staple cartridge body (1),
when the staple cartridge fastener (2) is in the second position of the staple cartridge fastener, a cutting edge or blade of the cutting assembly cuts through the staple cartridge biological patch (3), and the staple cartridge fastener (2) enables the front end of the staple cartridge biological patch (3) to be separated from the staple cartridge body (1).

11. The surgical stapler assembly according to claim 10, **characterized in that**, the staple cartridge fastener (2) is movably connected to the staple cartridge body (1) in the front-rear direction (X) of the staple cartridge body (1), and the staple cartridge fastener (2) is connected to the staple cartridge body (1) in both the first position of the staple cartridge fastener and the second position of the staple cartridge fastener,
the staple cartridge fastener (2) comprises two staple cartridge engagement protrusions (221), the front end of the staple cartridge biological patch (3) is provided with two staple cartridge biological patch engagement holes (31), and the two staple cartridge biological patch engagement holes (31) are located respectively on both sides of an extension line of the cutting edge or blade of the cutting assembly,
when the staple cartridge fastener (2) is in the first position of the staple cartridge fastener, the staple cartridge engagement protrusions (221) are inserted into the staple cartridge biological patch engagement holes (31), whereby the front end of the staple cartridge biological patch (3) is connected to the staple cartridge body (1),
when the staple cartridge fastener (2) is in the second position of the staple cartridge fastener, the staple cartridge engagement protrusions (221) are withdrawn from the staple cartridge biological patch engagement holes (31), whereby the staple cartridge biological patch (3) and the staple cartridge body (1) can be separated.

12. The surgical stapler assembly according to claim 1 or 10, **characterized in that**, the staple cartridge fastener (2) comprises an elastic retaining portion (23) of the staple cartridge fastener,
when the staple cartridge fastener (2) is in the first position of the staple cartridge fastener, the elastic retaining portion (23) of the staple cartridge fastener remains in its original state or undergoes slight deformation,
when the staple cartridge fastener (2) is in the second position of the staple cartridge fastener, the elastic retaining portion (23) of the staple cartridge fastener is compressed to generate elastic deformation or undergoes further elastic deformation.

13. The surgical stapler assembly according to claim 10, **characterized in that**, the staple cartridge body (1) is provided with a staple cartridge hook (11), a rear end portion of the staple cartridge biological patch (3) is provided with a staple cartridge biological patch socket hole (32), the staple cartridge hook (11) hooks into the staple cartridge biological patch socket hole (32), and the staple cartridge hook (11) comprises two separated portions, which are located on respectively both sides of the cutting edge or blade of the cutting assembly in a width direction (Y) of the surgical stapler assembly.

14. The surgical stapler assembly according to any one of claims 10 to 13, **characterized in that**, a staple cartridge biological patch slot (33) is provided at the front end of the staple cartridge biological patch (3), and extends to a front end edge of the staple cartridge biological patch (3) to form an opening, and when the staple cartridge fastener (2) is in the second position of the staple cartridge fastener, the cutting edge or blade of the cutting assembly moves to the staple cartridge biological patch slot (33) to cut off the staple cartridge biological patch (3).

15. A surgical stapler assembly, comprising a staple cartridge assembly (100) and an anvil assembly (200), the staple cartridge assembly (100) and the anvil assembly (200) being rotatably connected, **characterized in that**,
the anvil assembly (200) comprises an anvil body (4), an anvil fastener (5), and an anvil biological patch (6), the anvil body (4) is connected to a cutting assembly, and the cutting assembly can cut the anvil biological patch (6),
the cutting assembly can push the anvil fastener (5) in a front-rear direction (X) of the anvil body (4) to move between a first position of the anvil fastener and a second position of the anvil fastener,
when the anvil fastener (5) is in the first position of the anvil fastener, the anvil fastener (5) connects a front end of the anvil biological patch (6) to the anvil body (4),
when the anvil fastener (5) is in the second position of the anvil fastener, a cutting edge or blade of the cutting assembly cuts through the anvil biological patch (6), and the anvil fastener (5) enables the front end of the anvil biological patch (6) to be separated from the anvil body (4).

16. The surgical stapler assembly according to claim 15, **characterized in that**, the anvil fastener (5) is movably connected to the anvil body (4) in the front-rear direction (X) of the anvil body (4), and the anvil fastener (5) is connected to the anvil body (4) in both the first position of the anvil fastener and the second position of the anvil fastener,
the anvil fastener (5) comprises two anvil fastener engagement portions (51), and the front end of the anvil biological patch (6) is provided with two anvil biological patch engagement holes (61), which are located respectively on both sides of an extension line of the cutting edge or blade of the cutting assembly,
when the anvil fastener (5) is in the first position of the anvil fastener, the anvil fastener engagement portions (51) are inserted into the anvil biological patch engagement holes (61), whereby the front end of the anvil biological patch (6) is connected to the anvil body (4),
when the anvil fastener (5) is in the second position of the anvil fastener, the anvil fastener engagement portions (51) are withdrawn from the anvil biological patch engagement holes (61), whereby the anvil biological patch (6) and the anvil body (4) can be separated.

17. The surgical stapler assembly according to claim 15, **characterized in that**, the anvil fastener (5) comprises an elastic retaining portion (52) of the anvil fastener,
when the anvil fastener (5) is in the first position of the anvil fastener, the elastic retaining portion (52) of the anvil fastener abuts against the anvil body (4), whereby the anvil fastener (5) is prevented from moving forward,
when the anvil fastener (5) is in the second position of the anvil fastener, the elastic retaining portion (52) of the anvil fastener is compressed to generate elastic deformation.

18. The surgical stapler assembly according to claim 15, **characterized in that**, the anvil fastener (5) comprises a check portion, and the anvil body (4) comprises a blocking portion,
when the anvil fastener (5) is in the first position of the anvil fastener, the check portion is compressed and deformed by the blocking portion,
when the anvil fastener (5) is in the second position of the anvil fastener, the check portion moves to a front side of the blocking portion, whereby the blocking portion can prevent the anvil fastener (5) from moving from the second position of the anvil fastener to the first position of the anvil fastener.

19. The surgical stapler assembly according to claim 15, **characterized in that**, the anvil body (4) is provided with an anvil hook (41), a rear end portion of the anvil biological patch (6) is provided with an anvil biological patch socket hole (62), the anvil hook (41) hooks into the anvil biological patch socket hole (62), and the anvil hook (41) comprises two separated portions, which are located respectively on both sides of the cutting edge or blade of the cutting assembly in a width direction (Y) of the surgical stapler assembly.

20. The surgical stapler assembly according to any one of claims 15 to 19, **characterized in that**, an anvil biological patch slot (63) is provided at the front end of the anvil biological patch (6), and extends to a front end edge of the anvil biological patch (6) to form an opening, and when the anvil fastener (5) is in the second position of the anvil fastener, the cutting edge or blade of the cutting assembly moves to the anvil biological patch slot (63) to cut off the anvil biological patch (6).

21. A surgical stapler, **characterized in that**, the surgical stapler comprises the surgical stapler assembly according to any one of claims 1 to 19.
